Europäisches Patentamt

European Patent Office

Office européen des brevets

(19)

(11) EP 0 822 262 A1

(12) EUROPEAN PATENT APPLICATION

(43) Date of publication:
04.02.1998 Bulletin 1998/06

(51) Int Cl.⁶: **C13K 11/00**, C12P 19/14

(21) Application number: 97870111.8

(22) Date of filing: 25.07.1997

(84) Designated Contracting States:
AT BE CH DE DK ES FI FR GB GR IE IT LI LU MC NL PT SE
Designated Extension States:
AL LT LV RO SI

(30) Priority: 01.08.1996 BE 9600677

(71) Applicant: RAFFINERIE TIRLEMONTOISE
1150 Bruxelles (BE)

(72) Inventors:
• De Leenheer Leen
  3080 Tervuren (BE)
• Booten Karl
  3450 Geetbets (BE)

(74) Representative: Van Malderen, Joelle
Office Van Malderen,
Place Reine Fabiola 6/1
1083 Bruxelles (BE)

(54) **Process for producing a fructose syrup rich in fructose**

(57) The present invention is related to a process for producing a fructose syrup from fructan containing material by enzymatic hydrolysis in aqueous medium, wherein the fructan has an average degree of polymerisation of at least 17 and wherein directly, without any additional fractionating or refining, a syrup containing at least 93.5 weight percent fructose, calculated on dry substance, is obtained.

## Description

### Field of the invention

The present invention relates to a process for producing a fructose syrup which contains at least 93.5 weight percent, calculated on the dry substance, of fructose.

### Background and prior art

Fructose is a sugar which has a stronger sweetening power per weight unit than saccharose and can be used as sweetener in, for example, food, luxury foods, and pharmaceutical compositions. Besides, fructose has a higher solubility and is less viscous than saccharose.

It is known that classical methods, such as the enzymatic isomerisation of glucose obtained by hydrolysis from starch, or the acid or enzymatic hydrolysis of saccharose, yield syrups which contain maximally 50%, usually 40 to 50 %, fructose.

In view of the many advantages of fructose over glucose or saccharose, industry has always made large efforts to develop processes for producing fructose syrups containing more than 50 % fructose.

A classical way to obtain such syrups consists in the chromatographic separation of fructose from the other carbohydrates which are present in syrups obtained from saccharose or starch. The chromatographic separation technique is expensive and involves losses of fructose. As a result thereof, fructose syrups containing more than 50 % fructose are quite expensive.

Apart from the manufacture of fructose from saccharose or starch, fructose can also be prepared from fructan containing materials such as roots of chicory, tubers of Jerusalem artichoke, tubers of dahlia, and artichoke hearts of Globe artichoke.

Such process for producing fructose syrups rich in fructose from tubers of Jerusalem artichoke is described in the patent US-A-4,421,852. Inulin is extracted from the tubers of Jerusalem artichoke with warm water. The aqueous extract is concentrated and purified by ultrafiltration, then subjected to an enzymatic hydrolysis with inulinase. Accordingly, the hydrolysate is concentrated and purified by ultrafiltration. This process is yielding fructose syrups which contain at least 60 weight % of fructose. In the patent it is indicated that syrups can be obtained in this way which contain 75 to 90% of fructose, however no supporting example has been given.

A further method for preparing fructose syrups rich in fructose from fructan containing materials is described by L. De Leenheer in "Production and use of inulin : Industrial reality with a promising future" (Carbohydrates as Organic Raw Materials, Vol. III, 67-92 (1996)). The roots of chicory are treated with warm water to extract the inulin. The aqueous inulin extract obtained is then hydrolysed at 62 °C and at a pH of 4.5 by means of an inulinase (Fructozyme ® of NOVO), having endo- as well as exo-activity. The so obtained fructose syrup contains at least 85 % of fructose and is commercially available from ORAFTI (Belgium) under the name of Raftisweet ® F85/75 (ORAFTI product sheet 06/95).

To obtain syrups with a higher fructose content, chromatographic separation techniques have to be used, which are quite complex techniques thus involving higher product costs.

Such process is disclosed by Roquette Frères S. A. in FR-2618161, according to which a crude hydrolysate obtained by enzymatic hydrolysis of inulin extracted from chicory roots or from Jerusalem artichoke is subjected to a chromatographic fractionation on a cationic resin or on zeolites to yield a purified fructose syrup fraction containing 92 % fructose calculated on the total sugars content.

### Aims of the invention

The aim of the invention is to provide a process for producing fructose syrups rich in fructose, which does not have the disadvantages of the processes of the prior art and enables to produce such syrups in a much more simple and less expensive way compared to the prior art methods, from fructan containing material.

### Description of the invention

In accordance with the present invention , a process is provided for producing directly, without any additional fractionation or refining, a high purity fructose syrup containing at least 93.5 weight percent (weight %) of fructose, calculated on dry substance, involving enzymatic hydrolysis of a fructans containing material of which the fructans have an average degree of polymerisation of at least 17.

Applicants have indeed found that when a fructans containing material of which the fructans have an average degree of polymerisation of at least 17, is enzymatically hydrolysed, fructose syrups can be obtained directly, thus without any additional fractionation or refining, which contain at least 93.5 weight percent fructose, calculated on dry substance.

According to a preferred embodiment, the fructans of the fructan containing material have an average degree of polymerisation of at least 20.

In a further preferred embodiment, the fructose syrup obtained contains at least 94 weight % of fructose, preferably at least 95 weight % of fructose, calculated on dry substance.

In a more preferred embodiment, the average degree of polymerisation of the fructan is at least 23 and the fructose syrup obtained contains at least 94.5 weight %, preferably at least 95 weight % of fructose, calculated on dry substance.

In still a further preferred embodiment, the average degree of polymerisation of the fructan is at least 25 and the fructose syrup obtained contains at least 95 weight % of fructose, calculated on dry substance.

In fact, it can be stated that the higher the average degree of polymerisation, the higher the fructose content in the syrup which can be obtained.

It is advantageous to produce fructose syrups which contain at least 93.5 weight % fructose, preferably at least 95 weight % fructose, more preferably at least 96 weight % fructose. Such syrups are highly preferred in the world of diets.

By " fructan" is meant herein every compound wherein the majority of the bounds between the composing saccharide units consists of one or more fructosyl-fructose bounds, see "Glossary of fructan terms" (A.L. Waterhouse et al., Science and Technology of Fructans, p. 1-7 (1993)), incorporated herein by reference.

By "hydrolysis" is meant herein a "complete hydrolysis", to wit a hydrolysis wherein at the end of the reaction time the product to be hydrolysed is maximally split into monomers. In the case of fructans, this means that they are almost completely transformed into fructose (F) and glucose (G), and "end of the reaction time" is meant herein that moment in time from which the fructose content is not appreciably increasing anymore.

By degree of polymerisation (DP) is meant herein the number of saccharide units (fructose and glucose units) in one fructan molecule.

By average degree of polymerisation is meant the average degree of polymerisation ($\overline{DPn}$) calculated, after hydrolysis as follows :

$$\overline{DPn} = \frac{total\ \%\ F}{total\ \%\ G} + 1$$

The value is clearly a number average value. See in this respect the already cited article of L. De Leenheer, incorporated herein by reference.

Advantageously, fructans of the inulin or levan type are used in the process according to the invention. Inulin and levan essentially contain fructosyl-fructose bounds of the type beta-(2->1) and beta-(2->6), respectively (see L. De Leenheer, o.c.).

Inulin can occur as native inulin in plants or can be produced by micro-organisms. Native inulin usually has a degree of polymerisation ranging from 3 to 60 or even higher. The degree of polymerisation depends from the plant species, the age of the plant, the duration and conditions of the storage of the plant, as well as from the possible extraction process. Inulin can, for example, be extracted from chicory (Cichoreum intybus), tubers of dahlia (Dahlia variabilis), Jerusalem artichoke (Helianthus tuberosus) and artichoke hearts of Globe artichoke (Cynara scolymus). Inulin can possibly be extracted too from genetically modified plants. A method for genetically modifying plants has been described, for example, in WO94/14970. The average degree of polymerisation of fructans obtained from such plants can easily be more than 10,000.

An inulin which is suitable for use according to the present invention is, for example, an inulin with an average degree of polymerisation of 27 which is commercialised under the name SIGMA ® with reference I-2255, I-3754 and I-2880, according to the plant species, chicory, dahlia or Jerusalem artichoke , the inulin has been extracted from.

An other inulin suitable for use in the process according to the present invention, is inulin which has been extracted from roots of chicory and which is commercialised by ORAFTI under the name Raftiline ® HP. This product consists of at least 99.5% inulin with an average degree of polymerisation of at least 23 (see product sheet 04/96 issued by ORAFTI/Tiense Suikerraffinaderij (Belgium). A process for the manufacture of this product is described in patent application WO96/01849. According to this process, a solution of inulin is used as starting material which has been obtained by extraction at 65 °C of roots of chicory. The inulin solution is brought in a metastable phase, and accordingly cooled rapidly. Then the mass is seeded with inulin particles and after some time a fractionated inulin precipitate is formed in the solution. The precipitate is separated from the mother liquor by filtration, then washed and dried. The product obtained is free from impurities and has an average degree of polymerisation of at least 20, usually of at least 23. It is clear that the fractionated inulin which has been precipitated can be used immediately as such, after washing, in the process according to the present invention.

The average degree of polymerisation of inulin produced by micro-organisms can well reach 60,000. Such inulin is, for example, produced from saccharose in the presence of L-cysteine by Aspergillus sydowi conidia as described in the article "Characteristics and applications of a polyfructan synthesised from sucrose by Aspergillus sydowi conidia" (T. Harada et al., Food Hydrocolloids, Vol. 7 (1), 23-38 (1993)). The synthesis of bacterial inulin by means of fructosyltransferase from Streptococcus mutans has been described by J. Aduse-Opoku, FEMS Microbiology Letters, 59, 279-282 (1989) in the article "Genetic and antigenetic comparison of Streptococcus mutans fructosyltransferase and glucan-binding protein".

Levan is occurring in nature mainly in grasses, but is not produced at industrial scale from grasses. Levan is mainly obtained from synthesis by micro-organisms, for example, from saccharose by means of Bacillus subtilis levansucrase, as described in the article "Modification of the transfructosylation activity of Bacillus subtilis levansucrase by solvent effect and site-directed mutagens" (R. Chambert et al., A. Fuchs (Ed.), Inulin and inulin containing crops, p. 259 (1993)). Of course, levan can also be obtained by extraction from genetically modified plants, as indicated above for inulin.

The hydrolysis is carried out in aqueous medium, preferably in water, by subjecting the starting material to the action of the enzyme. Before subjecting it to hydrolysis, the fructan containing material is preferably brought in solution. Preferably, a solution is prepared containing 5-25 weight % dry substance (DS), more preferably from 10-20 % DS. It is most advantageous that the fructans are completely solubilised before subjecting them to enzymatic hydrolysis.

The complete, enzymatic hydrolysis per se , which can be carried out in neutral or slightly acidic medium, is well known in the art.

If the starting material is an inulin containing material, than preferably an enzyme preparation having inulinase activity and/or alpha-galactosidase activity is used. Such enzyme preparations are known and can be obtained, for example, from cultures of Penicillium, Aspergillus, Fusarium, Kluyveromyces, Candida and Sacharomyces. Preferably, the enzymatic hydrolysis is carried out according to the general method described by L. De Leenheer, o.c..

If the starting material is a levan containing material, than the hydrolysis is preferably carried out using an enzyme preparation having levanase activity, for example as obtained from cultures of Penicillium, Kluyveromyces or Saccharomyces.

Preferably, the enzymatic hydrolysis is carried out at a temperature of 58-64 °C and at a pH value ranging from 4 to 5. Usually, from 1 to 20 enzyme units (NOVO method) per gram fructan (inulin or levan) dry substance (DS) are added to the fructan solution, more preferably 2 to 3 enzyme units per gram fructan DS. The reaction time is than from 50 to 2.5 hours, and from 25 to 20 hours, respectively.

It is obvious that, in case fructans are hydrolysed having a fairly high average degree of polymerisation, for example higher than 50, in particular fructans originating from micro-organisms, it is advisable to increase, for example to double, the amount of enzyme units used in the hydrolysis, and/or to adapt (extend) the reaction time.

If required, the fructan syrup obtained from the hydrolysis can then be subjected to a demineralisation and a decoloration treatment, and the solution can optionally be further concentrated to yield a syrup with a defined content of dry substance. These techniques per se are well known in the art.

The present invention is illustrated by the examples given hereinafter describing a process for the manufacture of a fructose syrup containing at least 95 % fructose.

## Example 1

The starting material is inulin, extracted from roots of chicory, commercialised by ORAFTI (Belgium) under the trade name RAFTILINE ® HP. This inulin product contains more than 99.5 % inulin (calculated on dry substance) with an average degree of polymerisation of at least 23. First an aqueous solution of the inulin containing 15 weight % dry substance (DS) is prepared. Then the solution is brought at a pH of 8.5, the mixture is heated for 20 minutes at 100 °C to completely solubilise the inulin, and the clear solution obtained is cooled to 65 °C. Accordingly, the pH of the solution is reduced to 4.5 and 2.4 units Fructozymes ® from NOVO per gram inulin DS are added. The enzymes are allowed to react during 20 hours at a temperature of 60 °C, and at the end of the hydrolysis the solution is boiled for 2 minutes. The fructose syrup obtained is then demineralised and decolorised according to standard techniques. Finally, the syrup is further concentrated by evaporation of the water to yield a syrup with a concentration of 75 % dry substance.

The composition of the fructose syrup obtained has been analysed by HPLC (High Pressure Liquid Chromatography). The results are given in table 1 below. For comparison, the results of an HPLC analysis of a fructose syrup according to the art are also given in table 1. The comparative product is RAFTISWEET ® F85/75 which is commercialised by ORAFTI (Belgium).

Table 1

| | Fructose syrup obtained by the process of the invention | Fructose syrup obtained by the state of the art RAFTISWEET ® F85/75 |
|---|---|---|
| Polysaccharide | % carbohydrate on dry substance | |
| Fructose (F) | 95.1 | 85 |
| Glucose (G) | 4.4 | 13 |
| $F_2$ | 0.4 | < 1 |
| Saccharose (GF) | 0.1 | < 1 |
| DP > 3 * | 0 | < 1 |

*: DP : degree of polymerisation

## Example 2

The starting material is levan (from Erwinia herbicola, SIGMA ® L 8647). An aqueous solution of the levan (1 weight % dry substance) is prepared by boiling the product in water until it is fully dissolved (pH : 10.5 at 68 °C).

Then the solution is cooled to 60 °C, acidified with HCl 5N to a pH of 4.5, and accordingly 560 units enzyme (SP230 Fructozyme ® from NOVO) per gram dry levan substance are added to the mixture. The enzymes are allowed to act for 24 hours at 58-60 °C. The mixture is further processed as in example 1 above.

The composition of the fructose syrup obtained has been analysed by HPLC (High Pressure Liquid Chromatography). The results are given in table 2 below. For comparison, the results of an HPLC analysis if a fructose syrup according to the art are also given. The comparative product is RAFTISWEET ® F85/75 which is commercialised by ORAFTI (Belgium).

Table 2

| | Fructose syrup obtained by the process of the invention | Fructose syrup obtained by the state of the art RAFTISWEET ® F85/75 |
|---|---|---|
| Polysaccharide | % carbohydrate on dry substance | |
| Fructose (F) | 98.1 | 85 |
| Other saccharide DP 1 * | 1.7 | 13 |
| Saccharide DP 2 * | 0.2 | < 2 |
| Saccharide DP ≥ 3 * | < 0.4 | < 1 |

*: DP : degree of polymerisation

## Claims

1. Process for producing a fructose syrup from a fructan containing material by enzymatic hydrolysis in aqueous medium, characterised in that the fructan has an average degree of polymerisation of at least 17 and that directly, without a fractionation or refining, a fructose syrup containing at least 93.5 weight percent fructose, calculated on dry substance, is obtained.

2. Process according to claim 1, wherein the fructan has an average degree of polymerisation of at least 20.

3. Process according to claim 1, wherein the fructan has an average degree of polymerisation of at least 23 and a fructose syrup containing at least 94.5 weight percent fructose, preferably at least 95 weight percent fructose, calculated on dry substance, is obtained.

4. Process according to any of claims 1 to 3, wherein the hydrolysis is carried out in an aqueous medium containing 5 to 25 weight percent dry substance of the fructan containing material, at a pH of 4 to 5, at a temperature from 58 to 64°C, and using from 1 to 20 enzyme units (NOVO method) per gram dry substance of fructan.

5. Process according to any of claims 1 to 4 wherein the hydrolysis is carried out in an aqueous medium containing 10 to 20 weight percent dry substance of the fructan containing material using from 2 to 3 enzyme units (NOVO method) per gram dry substance of fructan.

6. Process according to any of claims 1 to 5, wherein the fructose syrup obtained after the hydrolysis is demineralised, decolorised and concentrated to 75 weight % dry substance.

7. Process according to any of claims 1 to 6, wherein the fructan is inulin and the enzyme preparation has inulinase activity and/or alpha-galactosidase activity.

8. Process according to any of claims 1 to 6, wherein the fructan is levan and the enzyme preparation has levanase activity.

9. Process according to any of claims 1 to 8, wherein the fructan is from plant origin, from genetically modified plant origin or produced by micro-organisms.

10. Process according to claim 9, wherein the fructan is inulin and the plant or genetically modified plant is chicory.

European Patent Office

## EUROPEAN SEARCH REPORT

Application Number

EP 97 87 0111

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | FR 2 504 939 A (NOVO INDUSTRI)<br>* page 2, line 5-12 *<br>* page 2, line 34-38 *<br>* page 3 *<br>* page 6, line 4-16 *<br>* page 9, line 35 - page 10, line 4 *<br>* page 14; table *<br>* claims; example 6 *<br>--- | 1-7,9,10 | C13K11/00<br>C12P19/14 |
| X | T.NAKAMURA ET AL.: "Continuous production of fructose syrups from inulin by immobilized inulinase from Aspergillus niger Mutant 817"<br>JOURNAL OF FERMENTATION AND BIOENGINEERING,<br>vol. 80, no. 2, 1995,<br>pages 164-169, XP002048211<br>* page 164, column 1, paragraph 1 *<br>* page 166, column 1, paragraph 3 *<br>* page 166, column 1, paragraph 5 - page 167, column 2, paragraph 1 *<br>--- | 1-5,7,9, 10 | |
| X | EP 0 177 477 A (REGION WALLONE)<br><br>* page 1, line 26-31 *<br>* page 7, line 4-6; claims 4-7; figure 4; example 2 *<br>--- | 1-5,7,9, 10 | TECHNICAL FIELDS SEARCHED (Int.Cl.6)<br><br>C13K |
| A | EP 0 011 350 A (STAMICARBON)<br>* the whole document *<br>--- | 1,6 | |
| A | FR 2 485 339 A (BEGHIN-SAY)<br>* the whole document *<br>----- | 1-10 | |

The present search report has been drawn up for all claims

| Place of search | Date of completion of the search | Examiner |
|---|---|---|
| THE HAGUE | 26 November 1997 | Van Moer, A |